# EUROPEAN PATENT SPECIFICATION

(11) **EP 3 975 836 B1**
(45) Date of publication and mention of the grant of the patent: **24.01.2024**
(21) Application number: 20726819.4
(22) Date of filing: 19.05.2020
(51) Int. Cl.: A61B 5/03, A61B 5/00, G01B 11/02, G01B 11/06, A61B 8/00, G01D 5/26

(54) **APPARATUS FOR MONITORING UTERINE CONTRACTIONS**
VORRICHTUNG ZUR ÜBERWACHUNG VON GEBÄRMUTTERKONTRAKTIONEN
APPAREIL DE SURVEILLANCE DES CONTRACTIONS UTÉRINES

(30) Priority: 27.05.2019 EP 19176778
(43) Date of publication of application: 06.04.2022
(73) Proprietor: Koninklijke Philips N.V., 5656 AG Eindhoven (NL)
(72) Inventor: YERMOSHKIN, Roman, 5656 AE Eindhoven (NL); GEYWITZ, Hansjoerg, 5656 AE Eindhoven (NL); WOHLSCHLAGER, Markus, Silvester, 5656 AE Eindhoven (NL)
(86) International application number: PCT/EP2020/063876
(87) International publication number: WO 2020/239513

(56) References cited:
- WO-A1-98/37807
- WO-A1-2018/029120
- US-A1- 2012 277 631

## Description

### FIELD OF THE INVENTION

The invention relates to an apparatus for monitoring uterine contractions.

### BACKGROUND OF THE INVENTION

Monitoring the uterine activity of a mother during labor and delivery is a standard measurement that usually accompanies the tracing of a fetal heart rate, which is often measured using ultrasound Doppler. In obstetrics, information about uterine contractions (such as a duration of a contraction, an intensity of a contraction, a waveform of a contraction) is important in the assessment of the wellbeing of a fetus during labor and delivery. For example, the interpretation of both the uterine activity and the fetal heart rate in a chronological sequence can assist medical personnel (such as doctors or caregivers) in providing the optimum treatment for the mother and the fetus.

A common technique for non-invasively deriving uterine contractions is to use a tocodynamometer (which may also be referred to as a toco). The tocodynamometer is placed on the abdominal wall of the mother and held in position with an elastic belt, which is looped around the belly of the mother. The tocodynamometer comprises a pressure sensor housed in a sensor housing. During a contraction of the uterus, tension changes of the uterine muscle occur and these changes are registered as a change in pressure force on a sensitive area of the pressure sensor, which is placed in the middle of the sensor housing. The sensitive area is surrounded by a stiff guard ring in order to reduce the influence of movement and breathing artefacts. A strain gauge element of the tocodynamometer translates the pressure force into an electrical signal.

However, while the strain gauge element is usually sensitive and stable, the strain gauge element is also complex to manufacture in terms of cutting, sputtering, etching, coating and final trimming. Due to this high manufacturing complexity, the strain gauge element is prone to quality variations. The strain gauge element is also expensive. Furthermore, due to the complex design of a strain gauge element, there are also size and placement restrictions associated with the strain gauge element.

US 3,945,373 discloses another type of tocodynamometer for providing an electrical output related to body surface displacement of a patient. The tocodynamometer comprises a light interrupter that extends into a space between an emitter and detector to interrupt light passing from the emitter to the detector, where uterine contractions cause displacement of the light interrupter and change the amount of light from the emitter that is received by the detector. However, this tocodynamometer is also complex to manufacture and is also mechanically complex. Moreover, the components of this tocodynamometer are not standard components and thus need to be specially manufactured. The components required for this tocodynamometer also occupy a large space (or volume), which means that the size of the tocodynamometer can be undesirably large.

US 2012/277631 describes a further example tocodynamometer. This example also uses an optical displacement sensor. A light emitter and an optical transducer is provided, as well as a reflector surface arranged to reflect at least part of the light emitted by the light emitter to the optical transducer. The reflector is coupled to a displacement member which contacts the mother's abdomen in use and moves responsive to movement of the abdomen. The movement modifies an intensity distribution of light reflected to the optical transducer.

This arrangement however is also structurally complex. In particular, the device requires that the angle of incidence of light at the reflecting surface to remain constant, and that only the point of incidence move. Displacement is detected by detecting movement in the location of the peak of the intensity distribution received at the optical transducer. Providing the displacement member so as to be moveable, while at the same time keeping a constant angle with respect to the light emitter and the optical transducer (i.e. the same incidence angle) increases the complexity of the device. In particular, a special housing must be provided which places the light emitter and optical transducer on different non-parallel surfaces. This also places constraints on the form factor and overall dimensions of the apparatus.

Furthermore, since the light incidence angle at the reflector surface is constant, and displacement is measured by measuring movement of the intensity distribution peak across the optical transducer surface, a complex transducer component must be provided. In particular, it must be capable of measuring variation of an optical intensity distribution across an extended area. This requires either an array of photodiodes, or a more complex photodiode. This increases cost compared to a device capable of using a single simple photodiode.

It would be beneficial to provide a contraction monitoring device having a simpler construction, and comprising simpler components.

### SUMMARY OF THE INVENTION

The invention is defined by the claims.

According to examples in accordance with an aspect of the invention, there is provided an apparatus for placement on the abdomen of a subject to monitor uterine contractions of the subject, the apparatus comprising:
a light source, and a light detector, and
a reflection surface facing the light source and light detector, arranged to provide optical communication between the two via reflection,
wherein one of the light source and light detector is fixed relative to the reflection surface, and the other is adapted to be moveable relative to the reflection surface responsive to uterine contractions, to thereby cause a variation in light intensity received at the light detector, the variation in light intensity being representative of uterine contractions of the subject.

Embodiments of the invention are based on measuring variation in magnitude of light intensity received at the light detector. Furthermore, embodiments are based on the concept of configuring the apparatus such that only one of the light source and light detector is moveable relative to a provided reflection surface, while the other is fixed. As a result of this, a simple arrangement can be provided wherein displacement between the moveable component and the reflector can be measured based simply on magnitude of light intensity received at the light detector. This arrangement does not require tracking of light intensity distribution meaning that a simpler light detector component can be provided, such as a simple photodiode.

Furthermore, the arrangement offers advantages even compared to other possible arrangements in which magnitude of light intensity is used as the parameter for detecting displacement. In particular, due to the fact that only one of the light source and light detector, moves relative to the reflection surface, a relationship between measured light intensity at the detector and displacement stays approximately linear over a longer displacement range. An approximately linear relationship is required for simple displacement determination. This means measurement range is increased. Furthermore, the linearity over a longer displacement range means that the measurement sensitivity remains constant.

By contrast, in possible examples not in accordance with the invention, in which both the light detector and light source are situated on one movable part, which moves relative to the reflection surface, the effect is that the displacement-intensity relationship stays linear for a short displacement period, but then tails off as both components approach nearer the reflector, since the incidence angle of the light source on the reflection surface either becomes too wide (in a case that the light source is the moveable one of the components), or too narrow (in a case that the detector is the movable one of the components), thus decreasing light intensity at the detector. Embodiments of the present invention avoid this.

The apparatus is arranged such that, when positioned in use on the abdomen of the subject, uterine contractions apply pressure to the moveable one of the light source and light detector to displace it relative the reflection surface. This alters an intensity of light received at the light detector. It is configured such that pressure exerted by the uterine contractions is coupled to the moveable part to displace it relative to the reflection surface by an amount depending on the force exerted by the abdomen of the mother. In this way, contractions can be determined, e.g. detected and/or measured.

The change in the intensity of light received at the light detector is as a consequence of the change in optical path length between the light source and light detector. It may also be caused in part by the change in angle of incidence of light on the reflection surface and at the light detector. In particular, the movement of the movable one of the light source and light detector responsive to the uterine contractions causes a change in the optical distance or displacement between the light source and light detector (and possibly also a change in angle of incidence of light on the reflection surface and at the light detector) and consequently causes a variation in the light intensity received at the light detector. It causes a change in the displacement between the movable one of the light source and light detector and the reflection surface, and this consequently causes the variation in light intensity received at the light detector. The variation in light intensity received at the light detector is dependent upon the change in relative positioning of the light detector and light source, in particular the change in optical path length induced between the two by the movement of the movable one of the two components.

The apparatus is arranged such that when the apparatus is placed in use on the abdomen of a subject, the movable one of the light source and light detector is arranged in direct or indirect contact with the abdomen. In this way the movement associated with the uterine contractions is coupled to the movable one of the light source and light detector.

Preferably, in use, the light source is configured to emit a light output of constant or uniform light intensity at least throughout the duration of a given use period. In this way, any variation in light intensity received at the light source is as a consequence of change in relative optical path between the light source and light detector (i.e. change in displacement between the movable one of the light source and light detector and the reflection surface).

The reflection surface is arranged facing the light source and the light detector. The reflection surface is positioned opposite the light source and light detector. The moveable one of the light source and light detector may be moveable in a direction towards and away from the reflection surface.

The apparatus may comprise a support structure or housing supporting or surrounding the light source, light detector and reflection surface. The support structure or housing is configured to be placed on the abdomen of the subject during use, such that the light source, light detector and reflection surface are all positioned in use on the abdomen of the subject, with the movable one of the light source and light detector arranged to move responsive to uterine contractions.

Preferably, the movement of the moveable one of the light source and light detector is such as to vary an angle of incidence of light at the light detector, e.g. an angle of incidence of light at a light input surface of the light detector.

In preferred examples, the movement is also such as to vary an amount of light flux received by the detector. The amount of light flux may change for example where the movement is such as to change a relative angle between the surface of the light detector and the reflection surface: this will alter an amount of light flux that falls incident on the detector from the reflection surface.

By varying the angle of incidence of the light on the light detector, the overall area over which the propagated light flux is spread on the detector surface varies. This thus changes the magnitude of the light intensity across the light sensitive area of the light detector as a function of displacement of the moveable component. Thus displacement can be measured based on detected light intensity at the detector.

In some cases, the movement is such as to vary the angle of incidence of light received at the light detector while keeping the angle of reflection of the light from the reflection surface toward the light detector constant, for example in cases in which the light detector is the moveable one of the components. However, this is not the case in all embodiments of the invention.

The moveable one of the light source and light detector may be moveable so as to vary an angle between the reflection surface and a surface of the light detector or the light source. For example, it may be moveable so as to vary an angle between the reflection surface and either a light input surface of the light detector or a light output surface of the light source. The angle here may refer to an angle in a direction perpendicular the reflection surface.

The facilitate this, the moveable one of the light source and light detector may in particular be arranged to be tiltable in a direction towards and away from the reflection surface. It may be tiltable away from a resting position towards the reflection surface.

Additionally or alternatively, the moveable one of the light source and light detector may be arranged to be moveable so as to increase or decrease an optical distance with respect to the reflection surface.

Optical distance means the optical path length between the reflection surface and the light source or light detector, e.g. the path length to a light output surface of the light source or a light input surface of the light detector.

According to one or more embodiments, each of the light source and light detector may be fixedly mounted on a separate respective substrate, one of the substrates being fixed relative to the reflection surface, and the other adapted to be moveable relative to the reflection surface. The movement of the moveable substrate thereby facilitates the movement of the moveable one of the light source and light detector.

Each of the substrates may be a PCB for example.

The moveable one of the substrates may be arranged to be tiltable in a direction towards and away from the reflection surface. Here, the substrate is adapted to be moveable in a tilting manner. An upper mounting surface of the substrate (to which the light source or light detector is mounted) may be tiltable relative to (in a direction towards or away from) the reflection surface.

The tilting of the substrate thereby varies an angle between the reflection surface and either a light output or input surface of the light source or light detector mounted on the substrate.

In certain examples, the tilting of the substrate may be in a direction away from the normal line of the reflection surface. In this case, the tilting is so as to tilt an upper surface of the light source or light detector in a direction away from the normal line as the component is moved from a resting position to a tilted position.

In advantageous examples, the at least a portion of the moveable substrate may be arranged to tilt about a tilt axis which is fixed in position relative to the reflection surface. It may be at least fixable relative to the reflection surface.

In advantageous embodiments, said at least portion of the moveable substrate may have a length extending in a direction away from the tilt axis, and a width extending parallel the tilt axis, and wherein the width of the portion varies along its length.

In preferred examples, the width may decrease with distance away from the tilt axis, e.g. as a function of distance from the tilt axis. The width may thus taper. The width may decrease monotonically. The width may decrease linearly or non-linearly.

In some examples, the tilt axis may be provided at a location across the body of the moveable one of the substrates. The tilt axis is formed in this case by the substrate itself.

Said moveable one of the substrates may incorporate an area portion which tilts about said tilt axis, the moveable one of the light source and light detector being mounted to the substrate on said area portion.

The substrate may incorporate a further area portion which does not tilt. It may be stationary. Alternatively it may tilt counter-directionally about the tilt axis for example.

In some examples, the tilt axis may be off-center relative to a plane defined by an upper mounting surface of the substrate, such that a larger area portion of the substrate tilts counter-directionally to a smaller area portion about the tilt axis. Preferably the one of the light source and light detector mounted to the substrate is mounted on the larger area portion.

According to any embodiment, the light detector and light reflector may be arranged adjacent one another, and in opposition to the reflection surface.

According to any embodiment, the reflection surface may be flat.

According to one or more embodiments, the apparatus may comprise a support structure having a contact portion arranged to rest against the abdomen of the subject in use, and wherein the reflection surface is fixed relative to the support structure, and the moveable one of the light detector and light emitter is moveable relative to the support structure.

In this way, the moveable element is configured to be displaced by movement of the abdomen, and configured to be displaced relative to the reflection surface which is held fixed to the support structure which rests solidly against the abdomen.

The apparatus may include a housing. The support structure may be a housing. The support structure may comprise a frame structure mounted within an outer housing.

The contact portion is at a base of the support structure, and the apparatus is placed on the abdomen via the contact portion. The contact portion is applied against the abdomen surface in use for example. The reflection surface may be spaced from the contact portion by a separation region. The moveable one of the light detector and the light source may be moveable in a direction towards and away from the reflection surface via said separation region.

According to one or more embodiments, the moveable one of the light detector and light emitter may be arranged to be biased toward a resting position by a biasing force.

It may furthermore be arranged such that when the apparatus is positioned in use on the abdomen of the subject, movement caused by uterine contractions exerts a counter-force against the biasing force to displace the light source or light detector away from the resting position.

The apparatus is arranged such that the movement caused by uterine contractions is coupled to the moveable one of the light source or light detector so as to cause said counter force to be applied. The apparatus may be arranged so that the movement of the abdomen directly applies or presses against the moveable component, or there may be a force transfer mechanism by which the force is indirectly coupled to the moveable component.

According to one or more embodiments, the light source, light detector and reflection surface may be coupled to a support structure, and wherein the apparatus comprises a belt for holding the support structure to the abdomen of the subject in use.

The support structure may be a housing, or be mounted within a housing, and wherein the housing is held against the abdomen in use by the belt.

According to one or more embodiments, the apparatus may further comprise a processor configured to:
receive an output signal from the light detector representative of an intensity of light received by the light detector over time, and
process the received output signal to determine uterine contractions based a variation of the intensity received by the light detector.

The processor may determine a variation of the intensity over time for thereby determining the uterine contractions.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a relationship between received light intensity and applied force for an example apparatus not according to the invention, in which the light source and light detector are both moveable;
Figs. 2a and 2b show one example apparatus according to one or more embodiments;
Fig. 3 further illustrates the example apparatus of Fig. 2;
Figs. 4a and 4b show a further example apparatus according to one or more embodiments;
Fig. 5 further illustrates the example apparatus of Fig. 4;
Fig. 6 shows a relationship between received light intensity and applied force for an example apparatus according to an embodiment;
Fig. 7 shows a plan view of the electrical layout out of components comprised by an example apparatus according to one or more embodiments;
Fig. 8 shows a plan view of an example apparatus according to an embodiment, and illustrates forces induced within the material of the substrate during use;
Fig. 9 shows a plan view of an example apparatus according to one or more embodiments comprising a moveable substrate with a tapered width;
Fig. 10 shows an illustration of an example apparatus placed on an abdomen of a subject to monitor uterine contractions according to an embodiment;
Fig. 11 shows a cross-sectional elevation view through an example apparatus comprising a support structure according to one or more embodiments;
Fig. 12 shows an underside view of an example apparatus according to one or more embodiments; and
Fig. 13 shows a block diagram of example processing steps for an output signal of a light detector comprised by an example apparatus according to one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for placement on the abdomen of a subject for monitoring uterine contractions. The apparatus includes a light source and a light detector which communicate light via a reflection surface arranged facing the light source and light detector. One of the light source and light detector is fixed in position relative to the reflection surface, while the other is arranged to be displaceable relative to the reflection surface responsive to movement of the subject's abdomen caused by contractions. The movement of the moveable component is such as to cause a variation in the light intensity received at the light detector.

By measuring light intensity variation over time, contractions can be detected and monitored. They may be quantified for example.

In certain preferable examples, the movement of the moveable one of the light source and light detector is such as to cause a variation in the angle of incidence of light received at the light detector. The movement may for instance be tilting movement of either the light detector or light source relative to the reflection surface. So long as one component remains fixed relative to the reflection surface, while the other moves, tilting of either component will lead to a change in the angle of incidence of reflected light on the light detector.

For brevity, the moveable one of the light source and light detector may be referred to generically in this disclosure as the 'moveable component' or 'moveable part'.

The provided arrangement offers advantages compared to other possible arrangements in which both the light source and light detector are moveable relative to the reflection surface. In particular, due to the fact that only one of the light source and light detector moves relative to the reflection surface, a relationship between measured light intensity at the detector and displacement of the moveable component remains approximately linear over a longer displacement distance. An approximately linear relationship is necessary for easily converting measured intensity into corresponding displacement.

This means possible measurement range is increased. Furthermore, the linearity over a longer displacement range means that the measurement sensitivity remains constant.

By contrast, in possible examples not in accordance with the invention, in which both the light detector and light source move relative to the reflection surface, the effect is that the displacement-intensity relationship remains linear for a short displacement period, but then tails off as both components approach nearer the reflector, since the incidence angle of the light source on the reflection surface either becomes too wide (in the case that the light source is the moveable one of the components), or too narrow (in the case that the light detector is the movable one of the components), thus decreasing light intensity at the detector.

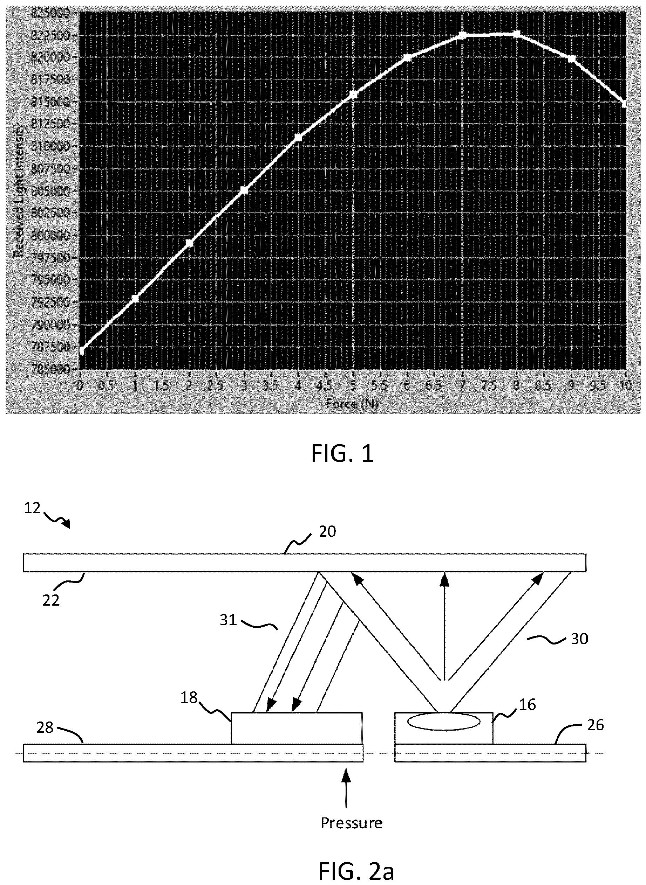

This is illustrated by the graph shown in Fig. 1 for example. This shows received light intensity (y-axis) at the light detector as a function of applied force for an arrangement in which both the light detector and the light source move together relative to the reflection surface. It can be seen that light intensity increases approximately linearly up to a certain applied force, but then tails downward beyond this force threshold. Thus, beyond this force threshold, reliable displacement measurement based on intensity is no longer possible.

Embodiments of the present invention aim among other things to avoid this. Thus, the maximum measurement force or maximum displacement range is increased. In particular, embodiments of the present invention provide the linear relationship over a much greater displacement distance, thus increasing maximum measurement force, as will be explained below.

Briefly, the apparatus provided herein is for placement on the abdomen of a subject to monitor uterine contractions of the subject. A uterine contraction is a hardening of the uterine muscle and causes a pressure force on the apparatus placed on the abdomen of the subject. According to some embodiments, the moveable part may be in direct contact with the abdomen of the subject when the apparatus is placed on the abdomen of the subject. Alternatively, according to some embodiments, the moveable part may be in indirect contact with the abdomen of the subject when the apparatus is placed on the abdomen of the subject.

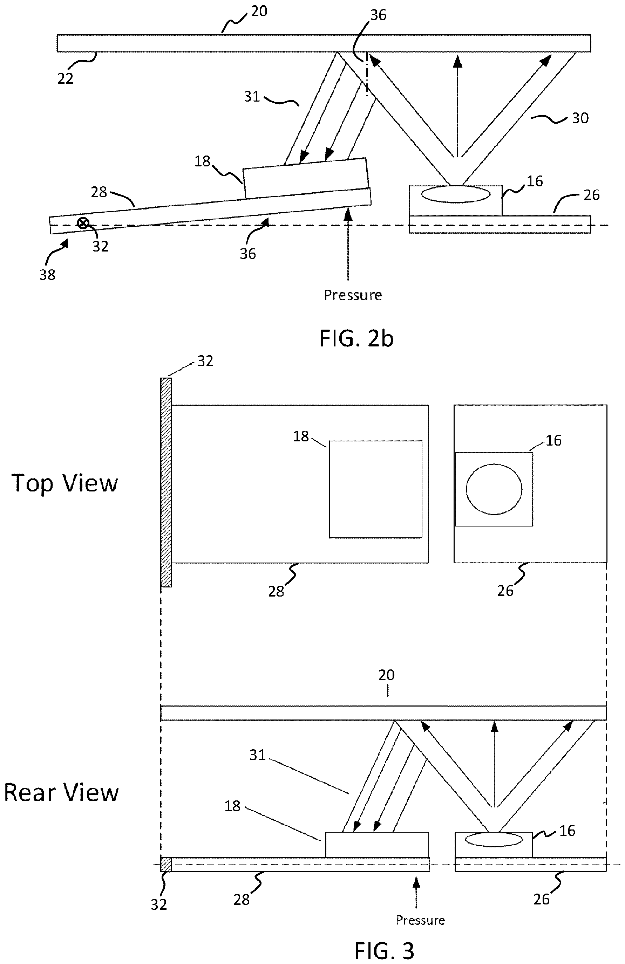

Figs. 2a and 2b schematically depict components of one example apparatus for monitoring contractions according to one or more embodiments of the present invention.

The apparatus 12 is for placement in use on the abdomen of a subject for monitoring the contractions. A housing or support structure may be provided surrounding or supporting the shown components. The illustrated components represent the optical arrangement of the device, for sensing and monitoring the contractions.

In brief, the apparatus 12 comprises a light source 16, and a light detector 18. In this example, the two are arranged adjacent one another. A reflection surface 22 is further provided, arranged opposite to and facing, the light source and light detector. The reflection surface is arranged to provide optical communication between the light source and light detector via reflection. In this example, a reflector plate 20 is provided, having a reflective lower surface 22 which provides said reflection surface. The reflection surface is preferably a specular reflective surface, but may be a diffuse reflective surface in other examples.

The reflection surface can be selected according to required reflectivity characteristics. The reflective properties of the reflective surface may be changed between different implementations of the apparatus, e.g. gold plated, tin plated, or coated with solder resist, to thereby change the reflective characteristics of the optical system. Since the reflective surface is spaced from the light source and light detector, the reflective properties of the surface may, if desired, be altered in this way very easily.

In use, light generated by the light source 16 is directed upward toward the reflective surface, where it is reflected and re-directed downward toward an sensitive upper surface of the light detector 18.

The light source 16, which may also be referred to as a light or optical transmitter, is configured to emit (or transmit) light 30. Examples of a light source include, but are not limited to a light bulb (for example, in combination with a lens), a light emitting diode (LED), or any other light source.

The light detector 18, which may also be referred to as a light or optical receiver, is positioned to detect (or receive) light emitted (or transmitted) from the light source 16 and then reflected 31 from the reflection surface 22. For example, a light sensitive area of the light detector can be positioned to detect reflected light from the reflection surface. Examples of a light detector include, but are not limited to a photodiode, a light dependent resistor (LDR) element, or any other light detector.

One of the light source 16 and light detector 18 is arranged to be fixed relative to the reflection surface 22, and the other is adapted to be moveable relative to the reflection surface responsive to uterine contractions. The movement of the moveable part is configured so as to cause a variation in light intensity received at the light detector 18. The variation in light intensity provides an indication of occurrence, and extent, of uterine contractions of the subject.

In particular, each of the light source 16 and light detector 18 is fixedly mounted on a separate respective substrate 26, 28. The substrates are PCBs in this example. One of the substrates is fixed relative to the reflection surface 22, and the other adapted to be moveable relative to the reflection surface. The movement of the moveable substrate facilitates the movement of the moveable one of the light source and light detector. The fixed configuration of the fixed substrate 26 ensures the fixed relationship between the fixed one of the light source 16 and light detector 18, and the reflective surface 22.

The fixed substrate 26 and moveable substrate 28 may be joined. The moveable substrate and fixed substrate may be different substrate portions of a single unitary substrate element. For example, the moveable substrate 28 may be formed by a partial cut-out portion of the single unitary PCB substrate, shaped to be bendable about a joining line with the remaining portion of the PCB. For example, the moveable substrate may be formed by a central bendable tongue portion of a single PCB substrate, the tongue portion joined across an end joining line to a surrounding outer annular frame portion, the frame portion forming the fixed substrate. This represents one example arrangement only.

In a further example arrangement, the fixed substrate 26 and moveable substrate 28 may be separate (non-joined) parts. They may extend for example from different sides of an outer housing structure for example. They may form different, separate, portions of a floor section of a housing for example.

In the particular example of Figs. 2a and 2b, the light source 16 is fixed relative to the reflection surface (via attachment to the fixed substrate 26), and the light detector 18 is arranged to be moveable relative to the reflection surface (via movement of the moveable substrate 28).

The movement of the moving part relative to the reflection surface in this example is constrained to a tilting, or cantilever, motion.

The light detector 18 is arranged to be tiltable in a direction towards or away from the reflection surface 22. In particular, the moveable substrate 28 to which the light detector is mounted is tiltable in a direction towards and away from the reflection surface 22, as illustrated in Fig. 2b, to thereby realize tilting of the light detector.

Thus, the movement of the moveable part is configured so as to vary an angle between an upper surface of the moveable one of the light source 16 and light detector 18 (e.g. light input surface of the light detector 18 or light output surface of the light source 16), and the reflective surface. This varies an incidence angle of reflected light upon the light input area of the light detector 18.

In use, the apparatus is placed on the abdomen of the subject, and is configured such that movements of the abdomen are directly or indirectly coupled to the moveable substrate 28 in the form of applied pressure, as illustrated in Fig. 2b. The applied pressure is coupled from the abdomen in a direction so as to cause tilting of the moveable substrate about a tilt axis 32 in a direction toward the reflection surface 22. A larger area portion 36 of the substrate 28 (to which the light detector 18 is mounted) is caused to tilt upward in a direction toward the reflective surface. This area portion 36 thus forms a lever portion of the substrate. It is noted that it is not essential that this moving lever portion be larger than other portion(s), although this may be preferred.

The amount of tilting may be a function of the applied pressure, such that as applied pressure increases, the substrate tilts further toward the reflective surface.

The moveable substrate 28 thus functions effectively as a lever member, exhibiting cantilever motion about the tilt axis 32 in response to applied pressure. The moveable substrate may be configured such that the extent of tilting (e.g. tilt angle or rotational displacement about the tilt axis 32) is proportional to the exerted force or pressure.

The tilting of the substrate 28 changes an angle between an upper light input surface (sensitive surface) of the light detector 18 and the reflective surface 22. This effectively results in a change in the angle of incidence of the reflected light 31 on the surface of the light detector 18. This in turn causes the light to be spread over a wider area across the surface of the light detector, reducing the incident light intensity as a function of tilting angle, which in turn is a function of applied force. Thus measured light intensity may be used to determine applied force and thus to determine contractions. The reduced light intensity is measurable at the light detector for example as a reduced photocurrent induced in the photodetector.

There are different options for the physical configuration of the substrates and the light source and light detector components within the apparatus.

As discussed above, in one example set of embodiments, the fixed substrate 26 and moveable substrate 28 may be joined. The moveable substrate and fixed substrate may be different substrate portions of a single unitary substrate element for example. For example, the moveable substrate may form a partially cut-away tongue portion of an overall unitary substrate structure. However, in other embodiments, the two substrates need not be joined; they may be separate substrate structures within the apparatus.

An example of the latter arrangement is shown schematically in Fig. 3. This depicts an aerial view of the example apparatus of Figs. 2a and 2b. The alignment of the side edges of the arrangement with those of the cross-sectional (or rear) view of the apparatus as depicted in Fig. 2 is also illustrated in Fig. 3, by virtue of dotted lines. As can be seen, in this example, the moveable substrate 28, to which the light detector 18 is mounted, and the fixed substrate 26, to which the light source 16 is mounted, are separate structures. The moveable substrate 28 is pivotably or bendably coupled to a fixed hinging or tilt axis 32. It is free around its three other side edges, meaning that it can pivot or bend about the hinging axis. This fixed hinging axis 32 may for example be a portion or section of a support structure (not shown) to which the arrangement of Fig. 3 is mounted. It may be a dedicated hinging beam. Alternatively, it may be provided by a side wall or surface of a support structure, such as a side wall of a housing structure of the apparatus.

Also shown in Fig. 3 is the fixed substrate 26, to which the light source 16 is mounted. This may be held fixed around all of its side edges, for example to a support structure or housing of the apparatus. Its side edges might be pinned for example to inner side edges of a housing or coupled to a rigid frame structure.

The fixed substrate 26 and moveable substrate 28 may extend for example from different sides of an outer housing structure. They may form different, separate, portions of a floor section of a housing for example.

Although in the example of Fig. 2, the two substrates are separate elements, in other examples, the two may form different portions of a single unitary substrate. Examples of this arrangement will be discussed in further detail later in this disclosure.

Although in the example of Fig. 2a and 2b, the light detector 18 is the moveable one of the light source 16 and light detector 18, arranged to be moveable relative to the reflection surface 22, in other examples the light source may instead be arranged to be moveable relative to the reflection surface, and the light detector fixed.

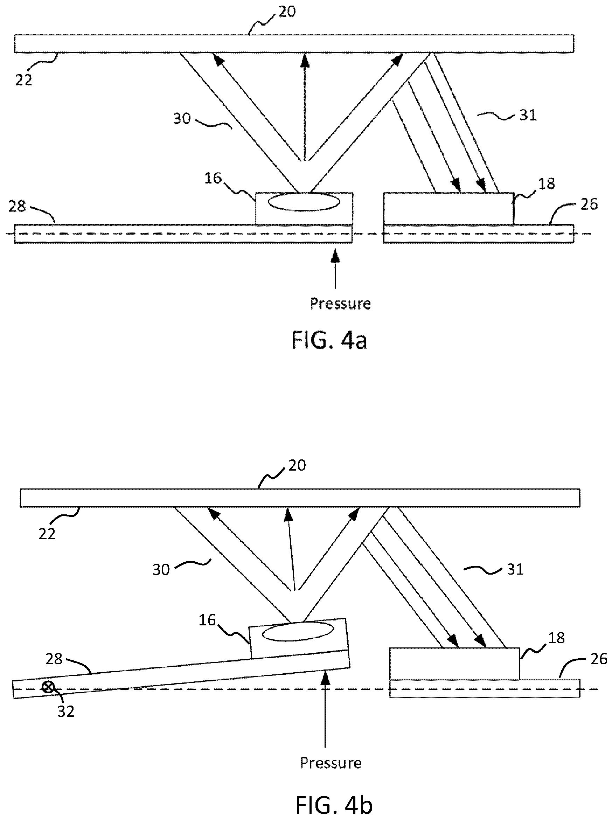

Figs. 4a and 4b show such an example.

Figs. 4a and 4b schematically depict a further example apparatus 12 in accordance with one or more embodiments, in which the light source 16 is arranged to be moveable relative to the reflection surface 22 and the light detector 18 is arranged fixed relative to the reflection surface. The arrangement of Figs. 4a and 4b is otherwise the same as that of Figs. 2a and 2b.

In particular, the light detector 18 is mounted to a PCB substrate 26 which is fixed relative to the reflection surface 22 and the light source 16 is mounted to a PCB substrate 28 which is moveable in a tilting fashion towards and away from the reflection surface about a tilt axis 32.

The apparatus 12 is arranged so that movement of the abdomen in use exerts a pressure which is coupled to the moveable substrate 28 in a direction so as to displace it about the tilt axis 32 toward the reflection surface 22. In particular, one area portion 36 of the substrate 28 (to which the light source 16 is mounted) is displaced in a tilting fashion upward in a direction toward the reflection surface 22. This is forms a lever portion 36 of the substrate. This is a larger area portion in this example.

As for the example of Figs. 2a and 2b, there are different options for the physical configuration of the parts of the apparatus of Fig. 4a and Fig. 4b.

In particular, in one example set of embodiments, the fixed substrate 26 and moveable substrate 28 may be joined. The moveable substrate and fixed substrate may be different substrate portions of a single unitary substrate element for example. For example, the moveable substrate may form a partially cut-away tongue portion of an overall unitary substrate structure. However, in other embodiments, the two substrates need not be joined; they may be separate substrate structure within the apparatus.

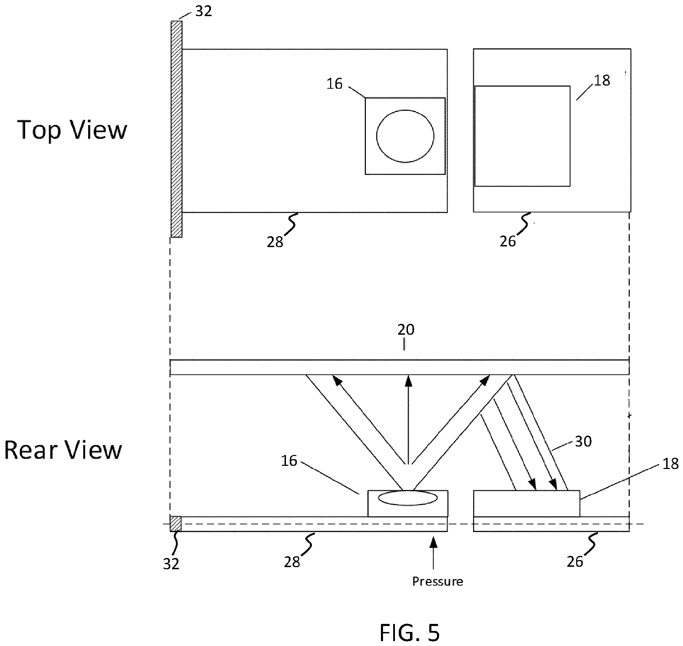

An example of the latter arrangement is shown schematically in Fig. 5. This depicts an aerial view of the example apparatus of Figs. 4a and 4b. The alignment of the side edges of the arrangement with those of the cross-sectional (or rear) view of the apparatus, as depicted in Fig. 4, is also illustrated in Fig. 5, by virtue of dotted lines. As can be seen, in this example, the moveable substrate 28, to which the light source 16 is mounted, and the fixed substrate 26, to which the light detector 18 is mounted, are separate structures. The moveable substrate 28 is pivotably or bendably coupled to a fixed hinging or tilt axis 32. It is free around its three other side edges, meaning that it can pivot or bend about the hinging axis. This fixed hinging axis 32 may for example be a portion or section of a support structure (not shown) to which the arrangement of Fig. 5 is mounted. It may be a dedicated hinging beam. Alternatively, it may be provided by a side wall or surface of a support structure, such as a side wall of a housing structure of the apparatus.

Also shown in Fig. 5 is the fixed substrate 26, to which the light detector 18 is mounted. This may be held fixed around all of its side edges, for example to a support structure or housing of the apparatus. Its side edges might be pinned for example to inner side edges of a housing or coupled to a rigid frame structure.

The fixed substrate 26 and moveable substrate 28 may extend for example from different sides of an outer housing structure. They may form different, separate, portions of a floor section of a housing for example.

Although in the example of Fig. 4 and 5, the two substrates are separate elements, in other examples, the two may form different portions of a single unitary substrate. Examples of this arrangement will be discussed in further detail later in this disclosure.

This changes an angle of the light output surface of the light source 16 relative to the reflection surface 22. Although it is the light source which is moving and changing angle, the effect is again effectively that the incidence angle of the reflected light 31 on the input surface of the light detector changes as a function of the degree of tilting of the moveable substrate 28. This is because the tilting of the light source results in a change in the light incidence angle at the reflective surface which, due to the law of reflection, results in an equal change in the angle of reflection of the light from the reflection surface. Since the light detector remains fixed relative to the reflection surface 22, this change in reflection angle results in an altered angle of incidence of the reflected light 31 upon the light input area of the light detector 18.

As explained above, this variation in light incidence angle at the light detector 18 leads to the incident light on the light detector 18 being spread over an increasing distance across the light detector surface which reduces an incident light intensity. This leads to a decrease in the photocurrent induced in a photodiode of the light detector for example.

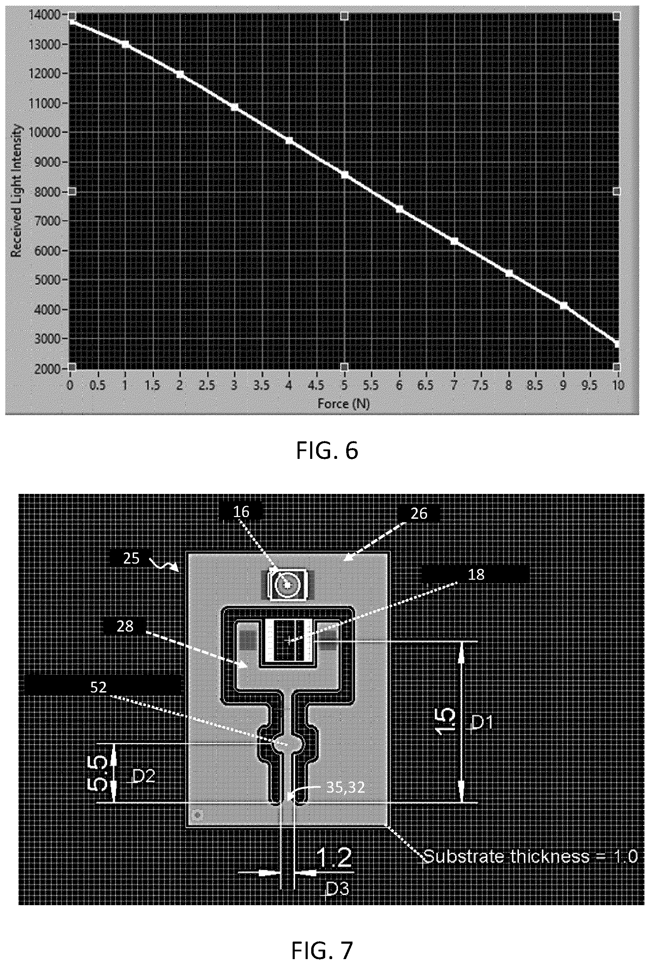

This effect is illustrated by the graph of Fig. 6. Fig. 6 shows the resulting measureable relationship between force exerted on the moveable one of the light source 16 and light detector 18 by movement of the abdomen during use (x-axis, Newtons), and the received light intensity at the light detector (y-axis; LSBs (least significant bit from A/D-Converter)). As shown, the relationship is approximately linear across a broad range of applied forces.

The shown relationship was achieved for an arrangement having dimensions wherein the maximum applied force (10 N) corresponded to a vertical displacement of the lever portion 36 of the moveable PCB 28 of approximately 1 mm.

The relationship is the same regardless of whether the light source 16 is arranged to be the moveable component or the light detector 18 is arranged to be the moveable component.

By measuring variation in the measured light intensity over time, applied force may in examples be determined and thus contractions detected and measured, e.g. quantitatively measured.

In the examples of Figs. 2-5 above, the moveable one of the substrates 26, 28 is arranged to tilt about a tilt axis 32 which is fixed relative to the reflection surface 22. This provides stable measurements.

However, this arrangement is not essential. In other arrangements for instance, the tilt axis 32 might be displaceable towards and away from the reflection surface. However, when in use, the tilt axis should at least be lockable in a fixed position relative to the reflection surface to provide stable measurements.

In the examples above, the tilt axis 32 is off-center relative to an upper mounting surface of the substrate 26, 28. One area portion 36 of the substrate may tilt counter-directionally to a further area portion 38 about the tilt axis, or the further area portion may be fixed or rigid, and the first area portion 36 tilts about the tilt axis, while the smaller area portion remains stationary. In each case, the one of the light source 16 and light detector 18 mounted to the moveable substrate 26, 28 is mounted on the first area portion 36.

The first area portion thus forms a lever portion 36 of the substrate, which tilts about the tilt axis 32.

In other examples however, the whole of the moveable substrate may tilt about the tilt axis. The whole substrate in this case forms a lever.

Increasing a length of the at least moveable (lever) portion 36 of the substrate enables a larger total displacement or tilting distance to be traversed by the moveable one of the light source 16 and light detector 18. This in turn increases a measurement sensitivity of the device. This will be discussed in more detail below.

Although in the above example, the moveable one of the light source 16 and light detector 18 is arranged to be tiltable in a direction towards or away from the reflection surface 22, in other examples a different mode of movement may be present. For example, the moveable component may be arranged to be moveable in a linear fashion towards or away from the reflective surface, i.e. to linearly decrease or increase an optical path length between the component and the reflective surface. Other examples will be apparent to the skilled person.

Although in the above examples, the movement of the moveable one of the light source 16 and light detector 18 is facilitated by movement of a substrate 26, 28 to which the component is mounted, this is not essential. In other examples, the components may be mounted in other configurations so as to facilitate movement of one component, and a fixed position of the other of the components. Various other arrangements will be immediately apparent to the skilled person.

Using the PCB substrate(s) to provide the movement is a particularly convenient arrangement, since each of the light source and light detector is required to be served by input and/or output wiring tracks to carry power to the component or sensor signals away from the component. Hence, simultaneously using a provided PCB to route required wiring to each component and to facilitate the moveable and fixed positions respectively reduces the total number of required components, since a separate movement mechanism is not required for example.

According to any embodiment, the light source 16 of the apparatus 12 may be configured to emit any type of light suitable to be detected by the light detector 18. For example, in some embodiments, the light source 12 may be configured to emit infrared (IR) light and the light detector may similarly be configured to detect infrared (IR) light. Infrared light has the advantage for example that there is less possibility of interference or contamination from any stray visible light. There are also a large number of off-the-shelf IR light sources and detectors available in the current technological environment.

For example, the light source 16 may be configured to emit light at a wavelength between 700nm and 1 µm (for example, at or approximately at 880nm, 850nm, 900nm, 950nm, or 1000nm). In some embodiments, where the light detector is an infrared photodiode, the light source may be configured to emit light at, or approximately at, a wavelength of 880 - 900 nm since infrared photodiodes tend to have their highest sensitivity around this wavelength.

As discussed, the mechanical displacement of the moveable one of the light source 16 and light detector 18 relative to the reflection surface 22 causes an intensity of the light that is detected by the light detector to vary. The intensity of the light that is detected by the light detector varies as a function of the movement of the moveable part, and this variation in the intensity of the light may be taken as representative of uterine contractions of the subject.

For example, the greater the uterine contraction of the subject, the greater the movement of the moveable part relative to the reflection surface, and thus the greater the variation in intensity of the light. Similarly, the smaller the uterine contraction of the subject, the smaller the movement of the moveable part, and thus the smaller the variation in intensity of the light.

According to one or more embodiments, it may be assumed that the variation in the intensity of the light is a function of the uterine contractions of the subject. According to some embodiments, a decrease in the light intensity may be indicative of the uterus contracting, while an increase in the light intensity can be indicative of the uterus relaxing. This is the case for the example arrangements of Fig. 2-3 and 4-5 discussed above for example.

However, in alternative example arrangements, an increase in the light intensity may be indicative of the uterus contracting, while a decrease in the light intensity can be indicative of the uterus relaxing.

In some embodiments, a pre-defined function or algorithm may be used to relate the variation in the intensity of the light to the uterine contractions of the subject. For example, a least squares algorithm may be used to determine a function for a regression line fitting a set of data points for the intensity of light detected by the light detector.

In any of the embodiments described herein, the intensity of the light that is detected by the light detector is converted into an electrical signal (such as a current or voltage variation). This may be received by a further processor component for example. It may be processed by the processor, e.g. to determine the uterine contractions. Processing methods analogous to those known from existing tocodynamometers may be used for this purpose for example, i.e. to relate the electrical signal to corresponding uterine contractions. The electrical signal in existing tocodynamometers is translated from a variation in measured pressure rather than light intensity, but a similar relationship exists.

The variation in the intensity of the light may be detected as a variation in a photocurrent induced in the light detector. The photocurrent is a function of the intensity of the light received at the light detector. Thus, in these embodiments, the photocurrent in the light detector is function of the uterine contractions of the subject.

According to any of the embodiments described herein, during uterine contractions, movement of the abdomen is coupled to the apparatus so as to cause movement of the moveable one of the light source 16 and light detector 18 relative to the reflection surface 22. According to certain examples, the maximum mechanical displacement of the moveable one of the light source and light detector may be configured to be equal to, or approximately equal to, 1mm.

According to any embodiment, the moveable one of the light source 16 and light detector 18 may be arranged to be biased toward a resting position by a biasing force, and arranged such that when the apparatus is positioned in use on the abdomen of the subject, movement caused by uterine contractions exerts a counter-force against the biasing force to displace the light source or light detector away from the resting position.

The force sensitivity of the apparatus depends upon the physical dimensions of various components. Most particularly, the sensitivity depends upon the physical dimensions of the moveable substrate 28 to which the moveable one of the light source 16 and light detector 18 is mounted.

The sensitivity refers to the smallest variation in applied force which the device is capable of distinctly measuring, i.e. the degree of precision to which the apparatus is able to measure applied force. Sensitivity increases as stiffness of the lever motion of the tilting substrate decreases, i.e. as the substrate becomes more flexible in its tilting action about the tilt axis 32.

Fig. 7 schematically illustrates the arrangement for an example fixed 26 and moveable 28 PCB substrate in accordance with one or more examples. The figure shows a plan-view of the layout of the two substrates.

As shown, in this example, the fixed PCB substrate 26 and moveable PCB substrate 28 are formed by different area portions of a single unitary PCB structure 25. The grey areas indicate the area of the PCB structure, while the black area represents the background. The moveable PCB substrate 28 is formed by an inner flexible tongue portion (a lever portion) of the overall PCB structure 25, the tongue portion partially cut away from the surrounding PCB structure to enable it is to flex up and down about an end pivot point 36 which defines a tilting axis 36 of the moveable PCB substrate.

The fixed PCB substrate 26 is formed by a surrounding frame portion of the overall PCB structure 25. The overall PCB structure is mounted in fixed relationship to the reflective surface in the assembled apparatus. This means that the surrounding frame portion of the structure 25 which forms the fixed PCB substrate 26 is fixed in position relative to the reflection surface 22, while the inner tongue (lever) portion forming the moveable PCB substrate 28 is moveable relative to the reflection surface by bending (i.e. tilting) out of plane of the PCB structure 25 about the pivot point, toward and away from the reflection surface.

In this example, the light source 16 is mounted on the fixed PCB substrate portion 26 and the light detector 18 is mounted on the moveable PCB substrate portion 28. However, as explained above, in other examples, these components may be mounted in the reverse configuration.

The substrate structure 25 may be arranged in the assembled apparatus such that pressure exerted in use upon the apparatus by uterine contractions is coupled to a particular force application area 52 or point on the moveable PCB substrate 28 area for thereby causing tilting movement of the moveable PCB substrate.

Example dimensions, in mm, for the certain parts of the arrangement are shown. Sensitivity of the apparatus can be altered by adjusting various of the shown dimensions.

For example, by increasing the distance, D2, between the force application point 52 of the moveable substrate portion 28, and pivot point 35 (the tilt axis 32) of the moveable substrate portion (i.e. the beginning point at which it joins the surrounding frame portion 26), the sensitivity increases. This occurs because the inner tongue portion forming the moveable substrate 28 becomes more flexible. Sensitivity increases indefinitely with increasing distance D2.

By increasing the distance, D1, between the force application point 52 and the position of the light detector 18 (or the light source 16 in further examples where the two components are switched), the sensitivity increases indefinitely, in relation to increasing D1.

The force sensitivity of the apparatus also has a reverse dependence on the width, D3, of the moveable substrate portion 28, and, separately, upon the substrate thickness. In particular, the narrower the width, D3, of the moveable substrate 28 and the thinner the PCB, the more sensitive is the achievable measurement.

Naturally, a trade-off is necessary between achievable sensitivity and physical dimensions. The possible dimensions of D1 and D2 are limited by the overall size of the device or structure which the apparatus is included within. Furthermore, the width D3 of the moveable substrate 28, and the substrate thickness also directly influence the mechanical stability and durability of the apparatus. Hence a further trade-off is necessary in this regard.

In experiments, a relation D1 : D2 : D3, which is approximately equal to 12.5 : 4.6 : 1 was found to be optimal during the experiments in terms of achievable force sensitivity and overall apparatus form factor. A measurement sensitivity of approximately 6 mN (Newtons⁻³) is achieved.

According to one or more embodiments, the moveable substrate portion 28 may be provided with a width which varies along the length of the portion. Preferably the width tapers along the length of the moveable substrate portion, decreasing as a function of distance from the tilt axis 32. This improves mechanical stability and robustness of the arrangement.

Tests have shown that a fixed width rectangular cantilever (i.e. a fixed with moveable substrate portion 28), has the following disadvantage. During displacement, the force is not distributed evenly over the material, but is concentrated at the base of the moveable substrate portion 28, at the area where the cantilever meets the tilt axis 32. This results in material degradation and the electric routes on the cantilever becoming worn or even being broken.

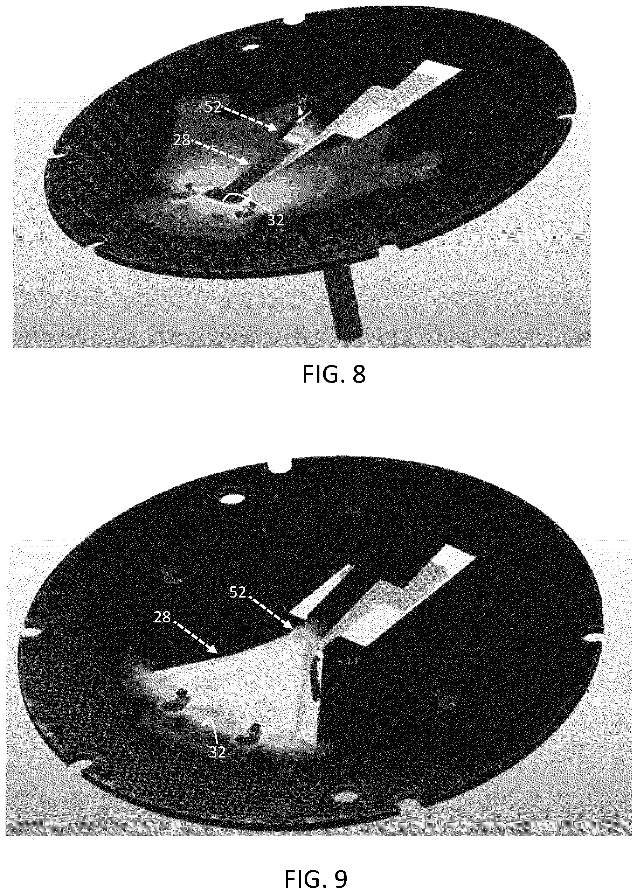

Fig. 8 shows a mechanical model of the substrate arrangement under stress. Darker colors correspond to greater mechanical stress, lighter colors correspond to lower stress. The figure shows the moveable substrate portion 28, which, as shown, forms a lever portion which is tiltable about the tilt axis 32. A force contact application area 52 for the moveable substrate 28 is also shown.

In the example model shown, the moveable substrate 28 is shown with a force of 10N being applied to the force application area 52. The model shows that by applying the force of 10 N in the middle of the substrate 28, the forces within the cantilever are very high, almost the whole way along the length of the lever (the substrate 28). In general, in order to avoid degradation of the material of the substrate, the elastic range of the material should not be exceeded during use. This requires that the forces encountered within the cantilever during normal use do not exceed the yield strength value of the material (which in typical examples is in the order of 70 MPa).

In the model shown in Fig. 7, the applied force of 10N leads to exceeding of the yield strength limit along the whole length of the moveable substrate portion 28 from the base of the substrate (where it meets the tilt axis 32) up to the force application point 52.

To address this problem, according to one or more embodiments, the moveable substrate 28 (or at least a portion of it which is configured to tilt) is provided having a width which varies along its length. In particular, the width tapers inwardly along the length of the substrate, decreasing with distance from the tilt axis. It may decrease along the portion of the substrate between the tilt axis and the force application point 52.

An example is shown in Fig. 9.

In this example, the moveable substrate 28 is again arranged tiltable about a tilt axis 32 at a base of the substrate. The substrate thus again forms a lever element. In this example, the moveable substrate 28 decreases in width along its length. The width in this particular example decreases between the tilt axis 32 and the force application point 52 of the substrate. However in other examples, the width may decrease over a longer section of the substrate.

In this example, the width tapers inwardly along the length of the substrate. The width may taper linearly (straight, inwardly sloping sides) or non-linearly (e.g. inwardly curving sides). For example, the width may decrease according to an exponential or logarithmic function of distance from the tilt axis 32 in some examples.

The mechanical stress model achieved using this example is shown in Fig. 9. Again, darker colors correspond to greater mechanical stress, lighter colors correspond to lower stress. For this example arrangement, the induced stress is lower across the whole of the substrate and in particular is low along the portion of the substrate over which the width decreases. The yield strength is not exceeded at any point along the substrate and the forces are distributed more evenly within the material:
The results obtained during long-term stress tests have shown similar results. Mechanical stability is significantly increased by the variable width adaptation.

In order to provide the tapering width shape, without decreasing measurement sensitivity, the overall arrangement may have to increase slightly in size compared to a rectangular cantilever arrangement. Hence a balance may need to be made between mechanical stability, size and measurement sensitivity.

Also, the achieved measurement curve (Fig. 6) may be less linear than with a rectangular cantilever example. Linearization may be performed during the signal processing to correct or improve this in some examples.

Although the above discussion has been made in relation to an example arrangement in which the moveable substrate 28 and fixed substrate 26 are different portions of a single unitary PCB structure, the same principles for configuring the apparatus can be applied to any other example arrangement (e.g. the arrangement schematically illustrated in Figs. 3 and 5 above). In particular, the details regarding configuring of the dimensions of the different substrates and positions of the light source and light detector can be applied to an arrangement in which the substrates are provided as separate elements, and separately coupled to a support structure or housing. The same principles regarding stress and substrate lengths apply equally to such an alternative arrangement.

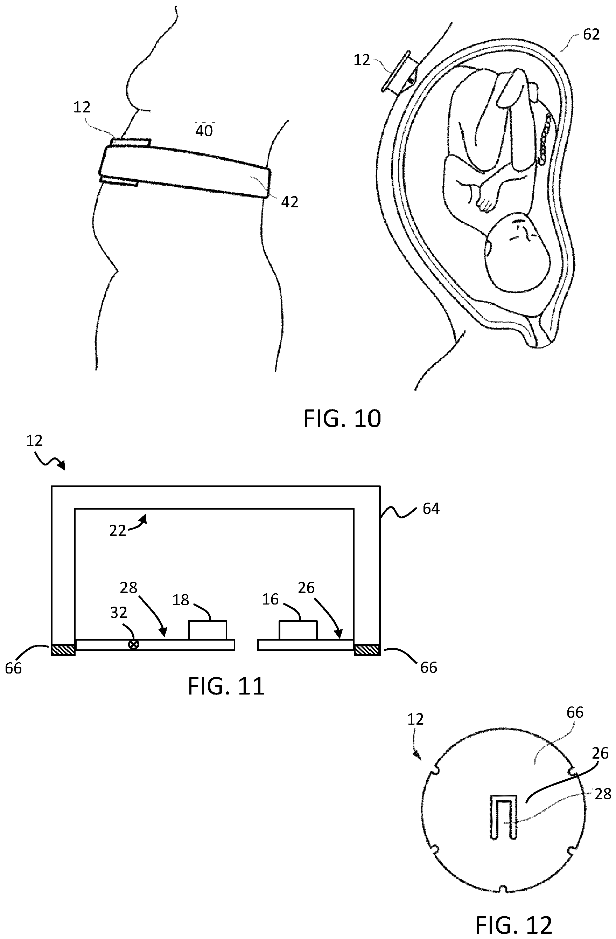

Fig. 10 illustrates an example apparatus 12 placed on an abdomen of a subject 40 to monitor uterine contractions according to one or more embodiments. In this illustrated example, a belt 42 is configured to be worn on the abdomen of the subject 40 to hold the apparatus 12 in place on the abdomen. The belt 42 is configured to extend around the torso of the body, at the abdomen. The belt 42 may for example be formed from, or may comprise, an elastic material. In some embodiments, the apparatus 12 may be placed between the belt 42 and the abdomen of the subject 40, as illustrated in Fig. 10. In this way, the belt 42 can be used to keep the apparatus 12 in place on (or fix the apparatus 12 to) the abdomen of the subject 40.

In examples, the apparatus may comprise a support structure, e.g. a frame or housing, for supporting components of the apparatus, and wherein the belt is arranged to hold the support structure in place on the abdomen of the subject during use.

In accordance with any embodiment described herein, the apparatus 12 may further comprise a housing configured to house the light source 16, the light detector 108 and the reflector element comprising the reflection surface 22.

In such embodiments, there may be no limitation regarding the wavelength (for example, the color) of the light that the light source 16 is configured to emit. In other words, the light source 16 may be configured to emit any wavelength (for example, any color) of light. This is possible where the light source 16 and the light detector 18 are contained within a housing (or a capsuled device) and thus not exposed to ambient light, which may otherwise lead to interference.

Nevertheless, as mentioned earlier, the light source 16 may in particular examples be configured to emit infrared (IR) light. Similarly, the light detector 18 may be configured to detect infrared (IR) light.

The apparatus 12 may according to one or more embodiments comprise a support structure 64 for supporting components of the apparatus for application in use to the abdomen.

Fig. 11 shows a cross-sectional elevation view through an example apparatus 12 comprising a support structure 64. The support structure may be a frame structure or a housing for example. The support structure has an contact portion 66 at its base, arranged to rest against the abdomen of the subject in use. The reflection surface 22 is fixed relative to the support structure. In this example, it forms an inner surface of the support structure for instance.

The moveable one of the light source 16 and light detector 18 is arranged moveable relative to the support structure. In this example, the light detector is mounted on a moveable PCB substrate 28 which is tiltable about a tilt axis 32. The tilt axis is secured fixedly relative to the support structure 64 (the attachment is not visible in this cross-sectional view). The light source is mounted on a further PCB substrate 26 which is fixed relative to the support structure. It is secured to the support structure. It may in some examples be formed by an outer annular section of an overall PCB structure, as in the examples of Figs. 7, 8, or 9, and the moveable PCB substrate may be formed by an inner tongue section of such a PCB structure for example. Alternatively, the moveable PCB substrate 28 and fixed PCB substrate 26 may be separate substrate structures, each coupled separately to the support structure 64, for example to the side walls of the support structure.

Fig. 12 shows a further example configuration for a housing or support structure of an apparatus according to one or more examples. An underside view of the underneath of the apparatus 12 is shown. As illustrated, in this example, the moveable substrate 28 is in the form of a flap anchored to a surrounding fixed PCB substrate 26. Hence, this arrangement is similar to that illustrated in Fig. 7 for example. The flap is moveable relative to the fixed part 26 responsive to uterine contractions of the subject. The surrounding fixed PCB part 26 also doubles as a contact portion 66 for the support structure.

For example, according to this embodiment, the moveable substrate part 28 may be formed by a milling groove in the fixed substrate part 26 that separates the moveable part 28 from the fixed part 26.

Figs. 11 and 12 represent examples only, and as will be appreciated by the skilled person, other possible structural arrangements and configurations consistent with the inventive concept are possible.

Although not illustrated in the figures, in any of the embodiments described herein, the apparatus 12 may further comprise a processor. The processor may control the operation of the apparatus 12. For example, the processor may be configured to control the light source 16 of the apparatus 12 to emit light and the light detector 18 of the apparatus 12 to detect the light emitted from the light source 16 via the reflection surface 22.

The processor may in examples comprise one or more processors, processing units, multi-core processors or modules that are configured or programmed to control the apparatus in the manner described herein. In particular implementations, the processor may comprise a plurality of software and/or hardware modules, each configured to operate, or which are for operating, the apparatus 12 described herein.

According to one or more embodiments, the processor may be configured to receive an output signal from the light detector 18 representative of the intensity of the light that is detected by the light detector 18. The processor may detect and monitor variation in the light intensity of the light over time based on the output signal. The processor may determine uterine contractions based on the determined variation of the intensity received by the light detector.

The received output signal provides a representation of the intensity of the light that is detected by the light detector 18 in the form of an electrical signal (such as a current variation or a voltage variation). For example, the pressure force dependent light intensity may be translated into a measurable voltage, which is then received by the processor as the output signal. The voltage values may be continuously measured by the processor. The processor may, for example, comprise an analog-to-digital (AD) converter to produce digitized values from the measured voltage values. As mentioned above, the processor may be configured to process the output signal received from the light detector 18 to determine uterine contractions based on a determined variation in the intensity of the light over time.

According to certain examples, the output signal (for example, the voltage values or the digitized voltage values) may be processed and filtered (for example, low-pass filtered to suppress artefacts such as from respiration) in the same way as is performed in a traditional tocodynamometer. For example, the processing may be performed in the same manner, with the exception that the voltage values are linearized and the gain and offset may be corrected in a different way.

In some embodiments, the processor may be configured to focus the processing of the received output signal on a frequency of a drive signal current with which the light source 16 is driven. The frequency of the signal with which the light source 16 is driven may be any frequency suitable for this purpose. In one example, the light source 16 may in examples be driven with a signal in the kilohertz (kHz) range. The light source 16 may be driven with any suitable current signal. In some embodiments, for example, the light source 16 can be driven with an alternating current (AC) signal. According to some embodiments, the light source 16 may be driven with a rectangular signal (for example, in the kHz range).

According to one or more embodiments, the processor may be configured to receive the output signal from the light detector 18 following pre-processing of the output signal at the light detector 18. Alternatively, pre-processing may be performed by the processor, e.g. a front-end of the processor.

For example, in some embodiments, the output signal received by the processor may be synchronously demodulated and filtered at the light detector 18. The synchronous demodulation and filtering may be performed with the same frequency or with a (for example, slightly) different frequency in order to transform a direct current (DC) signal to an alternating current (AC) signal. In this way, subsequent processing (such as amplification and noise filtering) is rendered more effective since the processing of an AC signal is more effective than the processing of a DC signal.

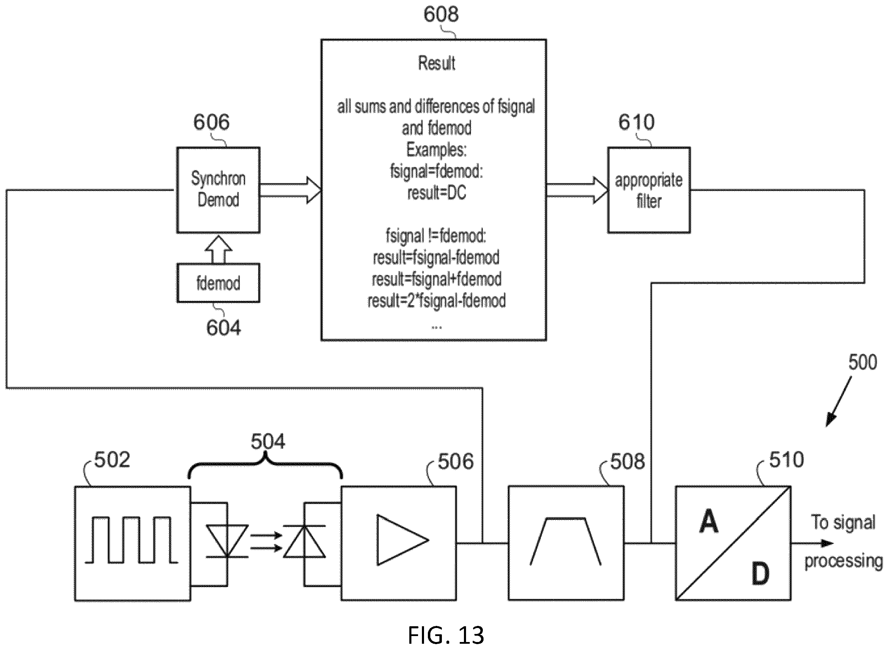

Fig. 13 shows a block diagram of example pre-processing stages for the output signal at the light detector 18 according to one or more embodiments. Alternatively, the same pre-processing steps may be performed by the processor.

As illustrated in Fig. 13, at stage 502, the light source 16 of the apparatus 12 is driven with a signal such that the light source 16 emits light, which can be detected by the light detector 18 of the apparatus 12. In this example embodiment, the light source 16 is driven with a rectangular signal (for example, in the kHz range). In this way, an opportunity is provided to adjust the optical path during final testing in the production process by varying the duty cycle. The light emitted by the light source 16 excites the light detector 18.

At stage 504, (for example, at the optical frontend of the light detector 18), the mechanical movement of the moveable one of the light source 16 and light detector 18 responsive to uterine contractions of the subject is converted into an electrical signal. More specifically, the mechanical movement causes an intensity of the light that is detected by the light detector 18 to vary, as explained above. This intensity variation over time, which is representative of the uterine contractions of the subject, is converted into an electrical signal. The electrical signal is an analog signal for example.

At stage 506, the electrical signal is amplified by an amplifier. As the light source 16 is driven with a rectangular signal (for example, in the kHz range) according to this example embodiment, 1/f noise of the amplifier is overcome, where *f* is the frequency of the output signal.

At stage 508, the amplified electrical signal is filtered. For example, the amplified electrical signal may be filtered using a bandpass filter. The bandpass filter may be tuned to the center of the excitation frequency of the light source 16.

According to one or more alternative embodiments, instead of the amplified electrical signal being filtered at stage 508, the amplified electrical signal may be passed to a synchronous demodulator. The amplified electrical signal has a modulation frequency f_{mod}. For example, the electrical signal may be modulated with a 1012 Hz carrier according to some embodiments. At stage 604, a demodulation frequency f_{demod} is set. For example, in some embodiments, the demodulation frequency f_{demod} may be set to 1 kHz. The electrical signal (or carrier) modulation frequency f_{mod} and the set demodulation frequency f_{demod} are synchronous and fixed, for example, by being coupled to a single frequency source.

At stage 606, the electrical signal is passed through the synchronous demodulator. The synchronous demodulator can, for example, comprise a simple switch followed by an integrator. At stage 608, processing is performed on the electrical signal to yield the electrical signal with a differential frequency f_{d}. The differential frequency f_{d} may, for example, be the difference between a modulation frequency f_{mod} of the electrical signal and a set demodulation frequency f_{demod} (i.e. f_{d} = f_{mod} - f_{demod}) where f_{demod} ≠ f_{mod}. For example, the differential frequency f_{d} may be set to 12 Hz.

At stage 610, the electrical signal is filtered such as by a bandpass filter. The bandpass filter may, for example, be tuned to 12Hz +/- 5 Hz. By setting the differential frequency f_{d} to 12 Hz, interference effects created by ambient light having a frequency at 50 to 60 Hz may be suppressed by setting the bandpass filter to 12 Hz +/- 5 Hz.

Whether stage 508, or stages 604 to 610, is followed, in either case, at stage 510, further signal processing may be performed on the filtered electrical signal. For example, this may involve the filtered electrical signal being passed through an analog-to-digital (A/D) converter, or through a rectification unit having a low pass filter and a subsequent analog-to-digital (A/D) converter. Then, the processor may be configured to receive the resulting signal as the output signal for processing in the manner described earlier to determine uterine contractions based on a determined intensity variation of the light that is detected by the light detector 18.

As mentioned above, the processor may be configured to focus the processing of the received output signal on the frequency of the signal with which the light source 16 is driven. In this way, interferences due to any interference effects can easily be blanked out.

In any of the embodiments described herein, the variation in detected light intensity over time, representative of the uterine contractions of the subject, may be compared to an acquired heart rate of the fetus of the subject over time. In these embodiments, the heart rate of the fetus of the subject may be acquired simultaneously with the variation in light intensity to enable a direct comparison to be made over a common time period. This comparison enables valuable information to be derived regarding on the relationship between the fetal heart rate and the uterine contractions.

Although the apparatus has been described herein as comprising a single light source and a single light detector, it will be understood that the apparatus may equally comprise a plurality of light sources and/or a plurality of light detectors that can operate in the manner described herein.

As discussed above, certain embodiments make use of a processor. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An apparatus (12) for placement on the abdomen of a subject to monitor uterine contractions of the subject, the apparatus comprising:
a light source (16), and a light detector (18), and
a reflection surface (22) facing the light source and light detector, arranged to provide optical communication between the two via reflection, and
wherein one of the light source (16) and light detector (18) is fixed relative to the reflection surface (22), and the other is adapted to be moveable relative to the reflection surface responsive to uterine contractions, to thereby cause a variation in light intensity received at the light detector, the variation in light intensity being representative of uterine contractions of the subject.

2. The apparatus (12) as claimed in claim 1, wherein the movement of the moveable one of the light source (16) and light detector (18) is such as to vary an angle of incidence of light at the light detector, and preferably is such as to vary an amount of reflected light that falls incident on the light detector.

3. The apparatus (12) as claimed in claim 1 or 2, wherein the moveable one of the light source (16) and light detector (18) is moveable in a direction towards or away from the reflection surface (22).

4. The apparatus (12) as claimed in any of claims 1-3, wherein the moveable one of the light source (16) and light detector (18) is moveable so as to vary an angle between the reflection surface (22) and a surface of the light detector or the light source.

5. The apparatus (12) as claimed in any of claims 1-4, wherein the moveable one of the light source (16) and light detector (18) is arranged to be tiltable in a direction towards or away from the reflection surface (22).

6. The apparatus (12) as claimed in any of claims 1-5, wherein the moveable one of the light source (16) and light detector (18) is arranged to be moveable so as to increase or decrease an optical distance with respect to the reflection surface (22).

7. The apparatus (12) as claimed in any of claims 1-6, wherein each of the light source (16) and light detector (18) is fixedly mounted on a separate respective substrate (26, 28), one of the substrates being fixed relative to the reflection surface (22), and the other adapted to be moveable relative to the reflection surface.

8. The apparatus (12) as claimed in claim 7, wherein the moveable one of the substrates (26, 28) is arranged to be tiltable in a direction towards and away from the reflection surface (22).

9. The apparatus (12) as claimed in claim 8 or claim 5, wherein the tilting is such as to tilt a surface of the moveable one of the light source (16) or light detector (18) in a direction away from a normal line (32) of the reflection surface (22).

10. The apparatus (12) as claimed in any of claims 7-9, wherein at least a portion of the moveable one of the substrates (26, 28) is arranged to tilt about a tilt axis (32) which is fixed relative to the reflection surface (22).

11. The apparatus as claimed in claim 10, wherein said at least portion of the moveable substrate has a length extending in a direction away from the tilt axis, and a width extending parallel the tilt axis, and wherein the width of the at least portion decreases with distance away from the tilt axis.

12. The apparatus (12) as claimed in any of claims 1-11, wherein the apparatus comprises a support structure having a contact portion arranged to rest against the abdomen of the subject in use, and wherein the reflection surface (22) is fixed relative to the support structure, and the moveable one of the light source (16) and light detector (18) is moveable relative to the support structure.

13. The apparatus (12) as claimed in any of claims 1-12, wherein the moveable one of the light source (16) and light detector (18) is arranged to be biased toward a resting position by a biasing force, and is arranged such that when the apparatus is positioned in use on the abdomen of the subject, movement caused by uterine contractions exerts a counter-force against the biasing force to displace the light source or light detector away from the resting position.

14. The apparatus (12) as claimed in any preceding claim, wherein the light source (16), light detector (18) and reflection surface (22) are coupled to a support structure, and the apparatus comprises a belt (42) for holding the support structure to the abdomen of the subject in use.

15. The apparatus (12) as claimed in any preceding claim, wherein the apparatus further comprises:
a processor configured to:
receive an output signal from the light detector (18) representative of an intensity of light received by the light detector over time, and
process the received output signal to determine uterine contractions based a variation of the intensity received by the light detector.

## Patentansprüche

1. Gerät (12) zur Platzierung auf dem Bauch eines Subjekts, um Uteruskontraktionen des Subjekts zu überwachen, wobei das Gerät umfasst: eine Lichtquelle (16) und einen Lichtdetektor (18) und eine Reflexionsfläche (22), die der Lichtquelle und dem Lichtdetektor zugewandt ist und so angeordnet ist, dass sie über Reflexion eine optische Kommunikation zwischen beiden ermöglicht, und wobei entweder die Lichtquelle (16) oder der Lichtdetektor (18) relativ zur Reflexionsoberfläche (22) fixiert ist und der andere relativ zur Reflexionsoberfläche als Reaktion auf Uteruskontraktionen beweglich ist, um dadurch eine Variation zu bewirken Lichtintensität, die am Lichtdetektor empfangen wird, wobei die Variation der Lichtintensität repräsentativ für Uteruskontraktionen des Subjekts ist.

2. Vorrichtung (12) nach Anspruch 1, wobei die Bewegung der beweglichen Lichtquelle (16) und des Lichtdetektors (18) derart ist, dass ein Einfallswinkel des Lichts am Lichtdetektor variiert wird Vorzugsweise ist es so, dass die Menge des reflektierten Lichts, das auf den Lichtdetektor fällt, variiert wird.

3. Vorrichtung (12) nach Anspruch 1 oder 2, wobei die bewegliche Lichtquelle (16) und der Lichtdetektor (18) in einer Richtung auf die Reflexionsfläche (22) zu oder von dieser weg beweglich sind.

4. Vorrichtung (12) nach einem der Ansprüche 1 bis 3, wobei die bewegliche Lichtquelle (16) und der Lichtdetektor (18) beweglich sind, um einen Winkel zwischen der Reflexionsfläche (22) und der Reflexionsfläche zu variieren eine Oberfläche des Lichtdetektors oder der Lichtquelle.

5. Vorrichtung (12) nach einem der Ansprüche 1-4, wobei die bewegliche Lichtquelle (16) und der Lichtdetektor (18) so angeordnet sind, dass sie in eine Richtung zur Reflexionsoberfläche hin oder von dieser weg geneigt werden können (22).

6. Vorrichtung (12) nach einem der Ansprüche 1-5, wobei die bewegliche Lichtquelle (16) und der Lichtdetektor (18) so angeordnet sind, dass sie beweglich sind, um einen optischen Abstand in Bezug darauf zu vergrößern oder zu verkleinern zur Reflexionsfläche (22).

7. Vorrichtung (12) nach einem der Ansprüche 1-6, wobei die Lichtquelle (16) und der Lichtdetektor (18) jeweils fest auf einem separaten Substrat (26, 28) montiert sind, einem der Substrate relativ zur Reflexionsfläche (22) fixiert ist und der andere relativ zur Reflexionsfläche beweglich ist.

8. Vorrichtung (12) nach Anspruch 7, wobei das bewegliche Substrat (26, 28) so angeordnet ist, dass es in eine Richtung zur Reflexionsfläche (22) hin und von dieser weg geneigt werden kann.

9. Vorrichtung (12) nach Anspruch 8 oder Anspruch 5, wobei die Neigung derart ist, dass eine Oberfläche der beweglichen Lichtquelle (16) oder des Lichtdetektors (18) in eine Richtung weg von einer Normalen geneigt wird Linie (32) der Reflexionsfläche (22).

10. Vorrichtung (12) nach einem der Ansprüche 7 bis 9, wobei mindestens ein Teil des beweglichen Substrats (26, 28) so angeordnet ist, dass es sich um eine Neigungsachse (32) neigt, die relativ dazu fest ist die Reflexionsfläche (22).

11. Vorrichtung nach Anspruch 10, wobei der zumindest Abschnitt des beweglichen Substrats eine Länge aufweist, die sich in einer Richtung weg von der Neigungsachse erstreckt, und eine Breite, die sich parallel zur Neigungsachse erstreckt, und wobei die Breite des zumindest Abschnitts nimmt mit der Entfernung von der Kippachse ab.

12. Gerät (12) nach einem der Ansprüche 1 bis 11, wobei das Gerät eine Stützstruktur mit einem Kontaktabschnitt umfasst, der so angeordnet ist, dass er im Gebrauch am Bauch des Subjekts anliegt, und wobei die Reflexionsfläche (22) fixiert ist relativ zur Stützstruktur, und das bewegliche Element aus Lichtquelle (16) und Lichtdetektor (18) ist relativ zur Stützstruktur beweglich.

13. Vorrichtung (12) nach einem der Ansprüche 1-12, wobei die bewegliche Lichtquelle (16) und der Lichtdetektor (18) so angeordnet sind, dass sie durch eine Vorspannkraft in eine Ruheposition vorgespannt werden Sie sind so angeordnet, dass, wenn das Gerät im Gebrauch auf dem Bauch des Subjekts positioniert ist, die durch Uteruskontraktionen verursachte Bewegung eine Gegenkraft gegen die Vorspannkraft ausübt, um die Lichtquelle oder den Lichtdetektor aus der Ruheposition zu verschieben.

14. Vorrichtung (12) nach einem der vorhergehenden Ansprüche, wobei die Lichtquelle (16), der Lichtdetektor (18) und die Reflexionsfläche (22) an eine Trägerstruktur gekoppelt sind und die Vorrichtung einen Gürtel (42) für umfasst Halten der Stützstruktur am Bauch des Probanden im Gebrauch.

15. Vorrichtung (12) nach einem der vorhergehenden Ansprüche, wobei die Vorrichtung weiterhin umfasst: ein Prozessor, der konfiguriert ist für: ein Ausgangssignal vom Lichtdetektor (18) zu empfangen, dass eine Intensität des vom Lichtdetektor empfangenen Lichts über die Zeit darstellt, und verarbeiten Sie das empfangene Ausgangssignal, um Uteruskontraktionen basierend auf einer Variation der vom Lichtdetektor empfangenen Intensität zu bestimmen.

## Revendications

1. Appareil (12) à placer sur l'abdomen d'un sujet pour surveiller les contractions utérines du sujet, l'appareil comprenant: une source de lumière (16) et un détecteur de lumière (18), et une surface de réflexion (22) faisant face à la source de lumière et au détecteur de lumière, agencée pour assurer une communication optique entre les deux via la réflexion, et dans lequel l'un de la source de lumière (16) et du détecteur de lumière (18) est fixe par rapport à la surface de réflexion (22), et l'autre est adapté pour être mobile par rapport à la surface de réflexion en réponse aux contractions utérines, pour provoquer ainsi une variation d'intensité lumineuse reçue au niveau du détecteur de lumière, la variation d'intensité lumineuse étant représentative des contractions utérines du sujet.

2. Appareil (12) selon la revendication 1, dans lequel le mouvement de la source de lumière (16) et du détecteur de lumière (18) mobile est tel qu'il fait varier un angle d'incidence de la lumière au niveau du détecteur de lumière, et est de préférence tel qu'il fait varier la quantité de lumière réfléchie qui tombe incidente sur le détecteur de lumière.

3. Appareil (12) selon la revendication 1 ou 2, dans lequel celui mobile parmi la source de lumière (16) et le détecteur de lumière (18) est mobile dans une direction vers ou loin de la surface de réflexion (22).

4. Appareil (12) selon l'une quelconque des revendications 1 à 3, dans lequel celui mobile parmi la source de lumière (16) et le détecteur de lumière (18) est mobile de manière à faire varier un angle entre la surface de réflexion (22) et une surface du détecteur de lumière ou de la source lumineuse.

5. Appareil (12) selon l'une quelconque des revendications 1 à 4, dans lequel celui mobile parmi la source de lumière (16) et le détecteur de lumière (18) est agencé pour être inclinable dans une direction vers ou loin de la surface de réflexion (22).

6. Appareil (12) selon l'une quelconque des revendications 1 à 5, dans lequel la source de lumière (16) et le détecteur de lumière (18) mobiles sont agencés pour être mobiles de manière à augmenter ou diminuer une distance optique par rapport à la surface de réflexion (22).

7. Appareil (12) selon l'une quelconque des revendications 1 à 6, dans lequel chacun parmi la source de lumière (16) et le détecteur de lumière (18) est monté de manière fixe sur un substrat respectif séparé (26, 28), l'un des substrats étant fixe par rapport à la surface réfléchissante (22), et l'autre étant adapté pour être mobile par rapport à la surface réfléchissante.

8. Appareil (12) selon la revendication 7, dans lequel celui mobile des substrats (26, 28) est agencé pour être inclinable dans une direction vers et loin de la surface de réflexion (22).

9. Appareil (12) selon la revendication 8 ou la revendication 5, dans lequel l'inclinaison est telle qu'elle incline une surface de la source de lumière (16) mobile ou du détecteur de lumière (18) dans une direction opposée à une direction normale (32) de la surface réfléchissante (22).

10. Appareil (12) selon l'une quelconque des revendications 7 à 9, dans lequel au moins une partie du substrat mobile (26, 28) est agencée pour s'incliner autour d'une ligne normale (32) qui est fixe par rapport à la surface de réflexion (22).

11. Appareil selon la revendication 10, dans lequel ladite au moins partie du substrat mobile a une longueur s'étendant dans une direction s'éloignant de l'axe d'inclinaison, et une largeur s'étendant parallèlement à l'axe d'inclinaison, et dans lequel la largeur de l'au moins partie diminue avec la distance par rapport à l'axe d'inclinaison.

12. Appareil (12) selon l'une quelconque des revendications 1 à 11, dans lequel l'appareil comprend une structure de support ayant une partie de contact agencée pour reposer contre l'abdomen du sujet utilisé, et dans lequel la surface réfléchissante (22) est fixée par rapport à la structure de support, et celui mobile parmi la source de lumière (16) et le détecteur de lumière (18) est mobile par rapport à la structure de support.

13. Appareil (12) selon l'une quelconque des revendications 1 à 12, dans lequel celui mobile parmi la source de lumière (16) et le détecteur de lumière (18) est agencé pour être sollicité vers une position de repos par une force de sollicitation, et est agencé de telle sorte que lorsque l'appareil est positionné en utilisation sur l'abdomen du sujet, le mouvement provoqué par les contractions utérines exerce une force contraire à la force de sollicitation pour éloigner la source de lumière ou le détecteur de lumière de la position de repos.

14. Appareil (12) selon l'une quelconque des revendications précédentes, dans lequel la source de lumière (16), le détecteur de lumière (18) et la surface de réflexion (22) sont couplés à une structure de support, et l'appareil comprend une courroie (42) pour maintenir la structure de support sur l'abdomen du sujet utilisé.

15. Appareil (12) selon l'une quelconque des revendications précédentes, dans lequel l'appareil comprend en outre: un processeur configuré pour: recevoir un signal de sortie du détecteur de lumière (18) représentatif d'une intensité de lumière reçue par le détecteur de lumière au fil du temps, et traiter le signal de sortie reçu pour déterminer des contractions utérines sur la base d'une variation de l'intensité reçue par le détecteur de lumière.
